# EUROPÄISCHE PATENTSCHRIFT

(11) **EP 0 712 833 B1**
(45) Veröffentlichungstag und Bekanntmachung des Hinweises auf die Patenterteilung: **03.09.1997**
(21) Anmeldenummer: 96101038.6
(22) Anmeldetag: 10.12.1991
(51) Int. Cl.: C07C 65/21

(54) **Verfahren zur Herstellung von 2-Phenoxymethylbenzoesäuren**
Process for the preparation of 2-phenoxymethylbenzoic acids
Procédé de préparation des acides 2-phénoxyméthylbenzoiques

(30) Priorität: 31.12.1990 DE 4042273; 31.12.1990 DE 4042280; 31.12.1990 DE 4042282; 31.12.1990 DE 4042283; 31.12.1990 DE 4042271; 31.12.1990 DE 4042272
(43) Veröffentlichungstag der Anmeldung: 22.05.1996
(62) Teilanmeldung aus: 91121148.0
(73) Patentinhaber: BASF AKTIENGESELLSCHAFT, 67056 Ludwigshafen (DE)
(72) Erfinder: Wingert, Horst, Dr., D-68159 Mannheim (DE); Wolf, Bernd, Dr., D-67136 Fussgönheim (DE); Benoit, Remy, Dr., D-67435 Neustadt (DE); Sauter, Hubert, Dr., D-68167 Mannheim (DE); Hepp, Michael, Dr., D-68526 Ladenburg (DE); Grammenos, Wassilios, Dr., D-67063 Ludwigshafen (DE); Kükenhöhner, Thomas, Dr., D-67459 Böhl-Iggelheim (DE)

(56) Entgegenhaltungen:
- CHEMICAL ABSTRACTS, vol. 115, no. 1, 8.Juli 1991, Columbus, Ohio, US; abstract no. 8314u, Seite 810, Spalte 1; & JP-A-2 306 934
- CHEMICAL ABSTRACTS, vol. 90, no. 1, 1.Januar 1979, Columbus, Ohio, US; abstract no. 6051x, Seite 6057, Spalte 2

## Beschreibung

Die vorliegende Erfindung betrifft ein Verfahren zur Herstellung von 2-Phenoxymethylbenzoesäuren der allgemeinen Formel IV 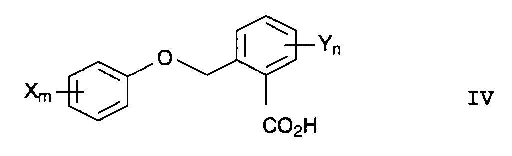 in der die Substituenten und die Indices die folgende Bedeutung haben:
- X,Y: Halogen, C₁-C₄-Alkyl, C₁-C₄-Alkoxy oder Trifluormethyl,
- m: eine ganze Zahl von 0 bis 4, wobei die Reste X verschieden sein können, wenn der Wert von m größer als 1 ist,
- n: eine ganze Zahl von 0 bis 3, wobei die Reste Y verschieden sein können, wenn der Wert von n größer als 1 ist.

Es ist allgemein bekannt, 2-Phenoxymethylbenzoesäuren durch Zusammenschmelzen von Phenolaten und Phthaliden herzustellen (vgl. z.B. Coll. Czech. Chem. Commun. 32, 3448 (1967), J. Chem. Soc., 4074 (1964), DE-A 22 08 893, DE-A 24 35 613 und US-A 4 282 365. Bei den genannten Verfahren werden primär Phenole mittels Base in die entsprechenden Phenolate überführt, wonach diese durch Abdestillieren des Lösungsmittels als Feststoff isoliert werden. Dieser Verfahrensschritt ist jedoch technisch nur schwer durchführbar, da das kristallisierende Phenolat mit abnehmender Lösungsmittelmenge immer schwerer durchmischt werden kann. Eine verläßliche Temperaturkontrolle ist daher nicht mehr möglich, so daß es zu starken Siedeverzügen kommen kann.

Ferner muß nach Zugabe des Phthalids zum Phenolat das Gemisch der beiden Feststoffe in aufwendiger Art und Weise in eine Schmelze überführt werden. Dabei treten Probleme bei der Durchmischung auf und es kommt sehr oft vor, dar größere Phenolatsalzbrocken in der Phthalidschmelze das Rühren beeinträchtigen bzw. zu einer Beschädigung des Rührers führen.

Ein weiterer Nachteil ist, daß bei den bekannten Herstellverfahren der Schmelzkuchen erst nach Abkühlen auf Raumtemperatur mit Wasser behandelt wird. Diese Vorgehensweise ist mit einem erheblichen Zeitaufwand verbunden. Zudem wird bei der Durchführung im technischen Maßstab ein Rührer mit variabler Eintauchtiefe erforderlich, der vor dem Erkalten aus der Schmelze herausgefahren werden kann oder es muß ein zweiter unabhängiger Rührer für den Auflösevorgang installiert werden.

Ein weiterer Nachteil der bekannten Herstellverfahren ist, daß die Säuren nicht mit ausreichender Reinheit anfallen, sondern in einem nachgeschalteten umkristallisationsschritt gereinigt werden müssen. Außerdem werden nur Ausbeuten von 60 - 70 % erreicht.

Gemäß der Lehre der DE-A 2 749 957 (Beispiel 6) und RO 78, 601 können 2-Phenoxmethylbenzoesäuren auch durch Umsetzung von Alkaliphenolaten mit Phthalid bzw. substituierten Phthaliden in Lösungsmitteln bei höheren Temperaturen hergestellt werden.

Diese Verfahrensweise hat jedoch den Nachteil, daß sehr lange Reaktionszeiten und große Lösungsmittelmengen erforderlich sind. Darüber hinaus ist das in der DE-A 27 49 957 beschriebene Herstellverfahren technisch recht aufwendig, weil aufgrund des Einsatzes von Natriumhydrid als Base und der Bildung von Wasserstoff bei der Reaktion hohe Sicherheitsvorkehrungen notwendig sind.

Der Erfindung lag daher die Aufgabe zugrunde, die Verbindungen IV besser zugänglich zu machen.

Demgemäß wurde ein Verfahren zur Herstellung von 2-Phenoxymethylbenzoesäuren der Formel IV gefunden, welches dadurch gekennzeichnet ist, daß man
a) ein Phenol der allgemeinen Formel II 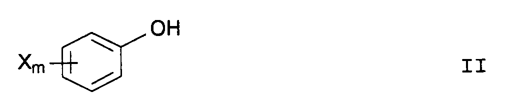 in Gegenwart eines Verdünnungsmittels mittels Base in das Phenolat überführt,
b) dieses anschließend mit einem Lacton der allgemeinen Formel III 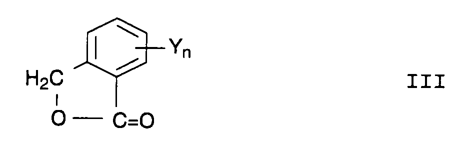 mischt,
c) das Verdünnungsmittel abdestilliert und das Gemisch in einem Temperaturbereich zwischen 5O und 250°C in der Schmelze umsetzt, und
d) die noch flüssige Schmelze mit Wasser auflöst und ansäuert.

Als Basen eigen sich insbesondere Alkalimetallhydroxide wie Natrium- und Kaliumhydroxid sowie Alkalimetall- und Erdalkalimetallalkoholate wie Natriummethanolat, Natriumethanolat und Kaliummethanolat; die Alkalimetallalkoholate sind besonders bevorzugt.

In der Regel führt man den Verfahrensschritt (a) in Gegenwart eines Lösungs- oder Verdünnungsmittels durch, wobei die Reaktionstemperatur normalerweise zwischen 0 und 100°C, bevorzugt zwischen 20 und 80°C liegt.

Als Lösungs- oder Verdünnungsmittel kommen aromatische Kohlenwasserstoffe wie Benzol, Toluol und o-, m-, p-Xylol, Alkohole wie Methanol, Ethanol und Isopropanol, Ether wie Tetrahydrofuran oder Gemische der genannten Lösungsmittel in Betracht. Besonders bevorzugt sind Methanol und Ethanol.

Die Menge an Base ist nicht kritisch. Für eine vollständige Überführung der Phenole II in die entsprechenden Phenolate werden mindestens äquimolare Mengen an Base benötigt; bevorzugt verwendet man einen Überschuß von 1 bis 6 mol-% an Base, bezogen auf die Menge an Lacton III.

Die Phenolate der Verbindungen II werden erfindungsgemäß ohne Isolierung aus der Reaktionsmischung mit den Lactonen der Formel III gemischt und, unter Entfernung des Lösungsmittels, zusammengeschmolzen.

Im allgemeinen führt man diesen Verfahrensschritt (c) bei einer Temperatur zwischen 50 und 250°C, vorzugsweise zwischen 160 und 220°C durch. Hierbei geht mit abnehmender Lösungsmittelmenge das Lacton III vom gelösten in den flüssigen Zustand über und man erhält eine gut durchmischbare Lösung des Phenolats im Lacton III.

Normalerweise setzt man Phenolat und Lacton in etwa stöchiometrischem Verhältnis ein, jedoch kann in manchen Fällen auch ein Überschuß der einen oder anderen Komponente, etwa bis zu 10 mol-%, empfehlenswert sein.

Bei den Verfahrensstufen (a) bis (c) sind keine besonderen Bedingungen bezüglich des Druckes erforderlich; zweckmäßigerweise arbeitet man daher bei Normaldruck.

Zur Beendigung der Reaktion verdünnt man die Schmelze vorteilhaft in noch flüssigem Zustand mit Wasser. Die erhaltene Lösung wird zur Freisetzung der 2-Phenoxymethylbenzoesäure IV angesäuert, bevorzugt mit einer anorganische Säure wie Salzsäure oder Schwefelsäure. Die weitere Aufarbeitung erfolgt wie üblich.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Die beschriebene Herstellungsmethode läßt sich mit Erfolg zur Synthese aller definitionsgemäßen 2-Phenoxymethylbenzoesäuren IV anwenden, vor allem auf solche Verbindungen, in denen die Substituenten X und Y jeweils ausgewählt sind aus einer Gruppe, bestehend aus:
- Halogen wie Fluor, Chlor und Brom, insbesondere Fluor und Chlor;
- verzweigtes oder unverzweigtes C₁-C₄-Alkyl wie Methyl, Ethyl, Isopropyl und n-Butyl, insbesondere Methyl und Ethyl
- C₁-C₄-Alkoxy wie Methoxy, Ethoxy, l-Methylethoxy und n-Propoxy;
- Trifluormethyl.

Die 2-Phenoxymethylbenzoesäuren der Formel IV werden überraschenderweise nach dem erfindungsgemäßen Verfahren in hoher Ausbeute und ausgezeichneter Reinheit zugänglich. Im Hinblick auf den bekannten Stand der Technik war dagegen zu erwarten, daß bei dem Verfahren die gleichen Schwierigkeiten auftreten, wie bei den bereits bekannten Herstellverfahren. Insbesondere war keinesfalls vorauszusehen, daß das Alkaliphenolat in Gegenwart des Lactons nach Abdestillieren des Verdünnungsmittels nicht als Feststoff ausfällt. Vielmehr hätte man eine zähe, schwer rührbare Reaktionsmischung mit Feststoffanteilen erwartet. Weiterhin war keineswegs zu erwarten, daß das Wasser problemlos bei der maximalen Reaktionstemperatur (ca. 200°C) zugetropft werden kann, ohne daß die Schmelze erstarrt.

Das erfindungsgemäße Verfahren besitzt gegenüber dem Stand der Technik eine Reihe von Vorteilen. Es ist im technischen Maßstab in sehr einfacher Weise durchführbar. Entscheidend ist, daß das intermediär erzeugte Alkali- phenolat nicht mehr als Feststoff isoliert werden muß, sondern daß ein fließender Übergang von der Lösung bzw. Suspension des Phenolats in die Schmelze (Phenolat + Lacton III) erfolgt. Dies wird durch Zugabe des Lactons III nach der Überführung des Phenols II, in Gegenwart eines Verdünnungsmittels, mittels Base in das Phenolat und anschließende Abdestillation des Verdünnungsmittels erreicht.

Ein zusätzlicher entscheidender Vorteil des erfindungsgemäßen Verfahrens besteht darin, daß der Schmelzkuchen nicht erkalten muß, sondern daß man bereits zur flüssigen Schmelze (etwa bei 200°C) Wasser unter Rückflußkühlung zugibt. Die Reaktionsmischung bleibt während der Verdünnung gut rührbar und die Temperatur sinkt mit zunehmender Wassermenge allmählich ab. Am Ende der Wasserzugabe liegt eine klare Lösung des Alkalisalzes der Säure IV vor. Die beim Ansäuern ausfallende Säure IV wird abgetrennt und mit wenig Wasser nachgewaschen.

Die 2-Phenoxymethylbenzoesäuren IV sind wertvolle Zwischenprodukte für die Synthese von E-Oximethern von Phenylglyoxylsäureestern I, die im Pflanzenschutz insbesondere als Fungizide Verwendung finden (vgl. EP-A 253 213 und der EP-A 254 426).

In folgendem Reaktionsschema ist ein besonders bevorzugter Syntheseweg zur Darstellung der Verbindungen I aus den 2-Phenoxymethylbenzoesäuren IV dargestellt.

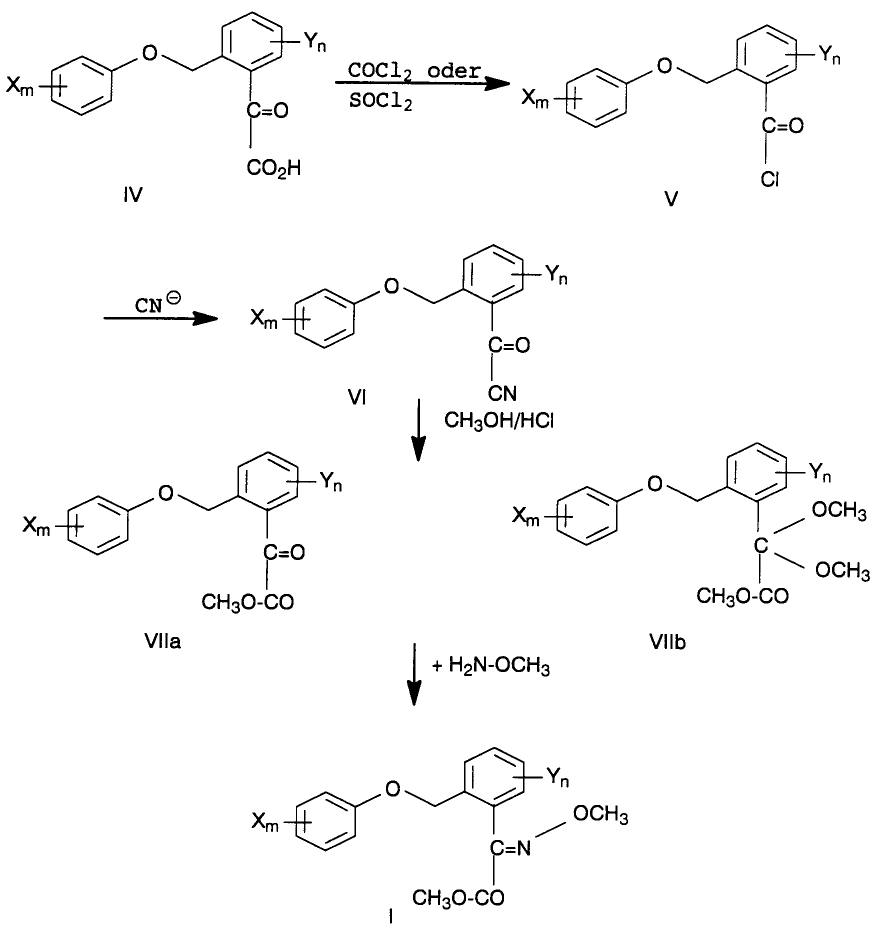

### Beispiele

### Beispiel 1

### 2-(2'-Methylphenoxymethyl)-benzoesäure

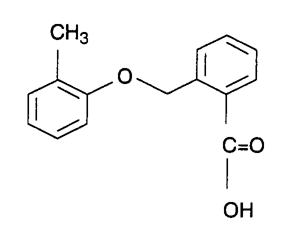

Zu 224 g (2,08 mol) o-Kresol wurden bei 35°C 382 g einer 30 gew.-%igen Lösung von Natriummethylat in Methanol (= 2,12 mol Natriummethylat) schnell zugetropft, wobei sich die Reaktionstemperatur auf 50°C erhöhte. Man rührte noch 1 Stunde bei dieser Temperatur, versetzte danach die Mischung mit 268 g (2 mol) Phthalid und destillierte anschließend das Methanol ab. Die dabei gebildete Schmelze wurde auf 200°C erhitzt, eine Stunde bei dieser Temperatur gerührt und dann langsam mit 1,6 l Wasser versetzt. Nach Erkalten wurde die erhaltene Lösung zweimal mit je 0,5 l Toluol extrahiert und dann mit 1 l Wasser verdünnt. Man säuerte mit konzentrierter Schwefelsäure an, bis die wäßrige Phase einen pH-Wert von 2 hatte, wonach der ausgefallene Rückstand abgetrennt, mit Wasser gewaschen und getrocknet wurde.
Ausbeute: 89 %; Fp.: 154°C
¹H-NMR (in CDCl₃; TMS als interner Standard): 2,35 ppm (3H); 5,55 ppm (2H); 6,88 ppm (2H); 7,15 ppm (2H); 7,42 ppm (1H); 7,63 ppm (1H); 7,87 ppm (1H) und 8,18 ppm (1H).

### Beispiel 2

### 2-Phenoxymethylbenzoesäure

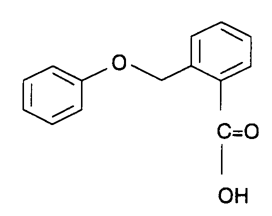

Zu 195,8 g (2,08 mol) Phenol wurden bei 45°C 382 g einer 30 gew.-%igen Lösung von Natriummethylat in Methanol (= 2,12 mol Natriummethylat) schnell zugetropft, wobei sich die Reaktionstemperatur auf 52°C erhöhte. Man rührte noch 1 Stunde bei 50°C, versetzte danach die Mischung mit 268 g (2 mol) Phthalid und destillierte anschließend das Methanol ab. Die dabei gebildete Schmelze wurde auf 200°C erhitzt, eine Stunde bei dieser Temperatur gerührt und dann analog Beispiel 1 auf das Produkt hin aufgearbeitet. Ausbeute: 86 %; Fp.: 132-133°C

### Beispiel 3

### 2-(Phenoxymethyl)-benzoesäure

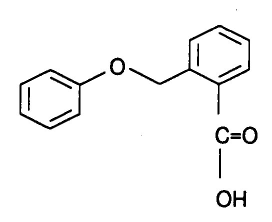

Eine Mischung aus 151 g (1,6 mol) Phenol, 106 g 85 gew.-%ige wäßrige Kaliumhydroxid-Lösung (entsprechend 1,6 mol KOH) und 1,5 l Xylol wurde unter laufender Entfernung des Wassers auf Rückflußtemperatur erhitzt. Nach Entfernung des Wassers versetzte man das Reaktiongemisch bei 100°C mit 201 g (1,5 mol) Phthalid und 57 ml Dimethylformamid und rührte anschließend noch 15 Stunden bei dieser Temperatur. Nach dem Abkühlen auf 20-25°C wurde das Produktgemisch zweimal mit je 2 l Wasser extrahiert und dann mit 140 ml 38 gew.-%iger wäßriger Salzsäure versetzt. Die gebildeten Kristalle wurden abgetrennt, mit 500 ml Wasser gewaschen und getrocknet. Zur Reinigung löste man das Rohprodukt in 550 ml warmem Acteon und fällte es durch Zugabe von 3 l Wasser wieder aus.
Ausbeute: 296 g; Fp.: 125-127°C;
¹H-NMR (in CDCl₃; TMS als interner Standard): 5,55 ppm (s,2H); 7,00 ppm (m,3H); 7,30 ppm (t,2H); 7,40 ppm (t,1H); 7,65 ppm (t,1H); 7,85 ppm (d,1H); 8,20 ppm (d,1H).

### Beispiel 4

### 2-[(2'-Methyl)-phenoxymethyl]-benzoesäure

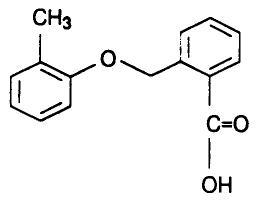

Eine Mischung aus 160 g (1,48 mol) o-Kresol, 106 g 85 gew.-%ige wäßrige Kaliumhydroxid-Lösung und 1,5 l Xylol wurden unter laufender Entfernung des Wassers auf Rückflußtemperatur erhitzt. Dann kühlte man die Mischung auf 100°C und versetzte sie bei dieser Temperatur mit 195 g (1,45 mol) Phthalid und 57 ml Dimethylformamid. Das erhaltene Gemisch wurde noch 15 Stunden auf 100°C erhitzt, anschließend auf 20-25°C abgekühlt, zweimal mit je 2 l Wasser extrahiert und dann mit 140 ml 38 gew.-%iger wäßriger Salzsäure versetzt. Die gebildeten Kristalle wurden abgetrennt, mit 500 ml Wasser gewaschen und getrocknet. Zur Reinigung löste man das produkt in 550 ml warmem Aceton und fällte es durch Zugabe von 3 l Wasser wieder aus. Ausbeute: 85 %; Fp.: 154°C;
¹H-NMR (in CDCl₃, TMS als interner Standard): 2,39 ppm (s,3H); 5,38 ppm (s,2H); 6,9-8,2 ppm (m,8H).

## Patentansprüche

1. Verfahren zur Herstellung von 2-Phenoxymethylbenzoesäuren der allgemeinen Formel IV 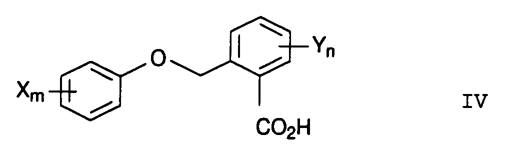 in der die Substituenten und die Indices die folgende Bedeutung haben:
X,Y Halogen, C₁-C₄-Alkyl, C₁-C₄-Alkoxy oder Trifluormethyl,
m eine ganze Zahl von 0 bis 4, wobei die Reste X verschieden sein können, wenn der Wert von m größer als 1 ist,
n eine ganze Zahl von 0 bis 3, wobei die Reste Y verschieden sein können, wenn der Wert von n größer als 1 ist,
dadurch gekennzeichnet, daß man ein Phenol der allgemeinen Formel II 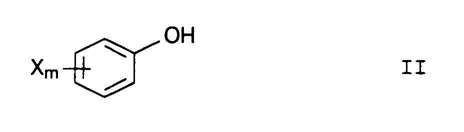 in Gegenwart eines Verdünnungsmittels mittels Base in das Phenolat überführt, dieses mit einem Lacton der allgemeinen Formel III 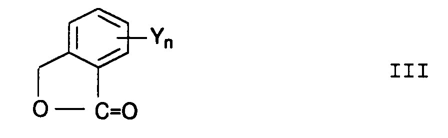 mischt, das Verdünnungsmittel abdestilliert und das Gemisch in einem Temperaturbereich zwischen 50°C und 250°C in der Schmelze umsetzt und die noch flüssige Schmelze mit Wasser auflöst und ansäuert.

## Claims

1. A process for preparing a 2-phenoxymethylbenzoic acid of the formula IV 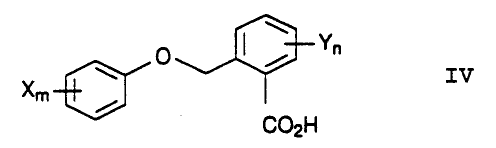 where
X and Y are each halogen, C₁-C₄-alkyl, C₁-C₄-alkoxy or trifluoromethyl,
m is an integer from 0 to 4, it being possible for the radicals X to be different when the value of m is greater than 1,
n is an integer from 0 to 3, it being possible for the radicals Y to be different when the value of n is greater than 1,
which comprises converting a phenol of the formula II 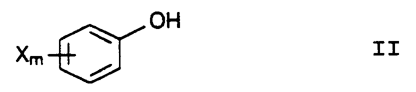 with a base into the phenolate in the presence of a diluent, mixing this phenolate with a lactone of the formula III 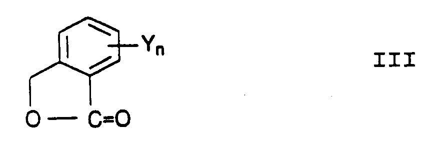 removing the diluent by distillation, and reacting the molten mixture at from 50°C to 250°C, and dissolving the melt while still liquid in water and acidifying.

## Revendications

1. Procédé de préparation des acides 2-phénoxyméthylbenzoïques de formule générale IV 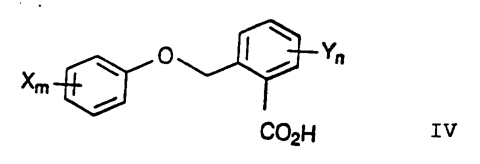 dans laquelle les symboles et les indices ont les significations suivantes :
X, Y : halogène, alkyle en C1-C4, alcoxy en C1-C4 ou trifluorométhyle,
m : un nombre entier allant de 0 à 4, les substituants X pouvant être différents lorsque m est supérieur à 1,
n : un nombre entier allant de 0 à 3, les substituants Y pouvant être différents lorsque n est supérieur à 1,
caractérisé par le fait que l'on convertit un phénol de formule générale II 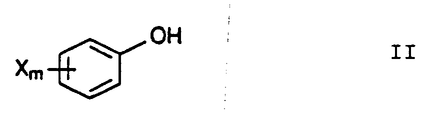 en présence d'un diluant et à l'aide d'une base, en le phénolate correspondant qu'on mélange avec une lactone de formule générale III 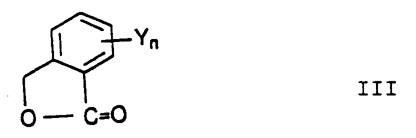 après quoi on distille le diluant et on fait réagir le mélange à l'état fondu dans l'intervalle de température allant de 50 à 250° C puis on dissout la masse fondue encore fluide dans l'eau et on acidifie.
